# EUROPÄISCHE PATENTSCHRIFT

(11) **EP 1 207 848 B1**
(45) Veröffentlichungstag und Bekanntmachung des Hinweises auf die Patenterteilung: **10.11.2004**
(21) Anmeldenummer: 00960537.9
(22) Anmeldetag: 28.08.2000
(51) Int. Cl.: A61K 7/48, A61K 35/80, A61K 33/12, A61K 33/14

(54) **ENTSCHLACKUNGS- UND/ODER REGENERATIONSMITTEL**
PURIFYING AND/OR REGENERATING AGENT
AGENT D'ELIMINATION ET/OU DE REGENERATION

(30) Priorität: 31.08.1999 DE 19941360
(43) Veröffentlichungstag der Anmeldung: 29.05.2002
(73) Patentinhaber: Seemann, Rüdiger, 81547 München (DE)
(72) Erfinder: Seemann, Rüdiger, 81547 München (DE)
(74) Vertreter: Strych, Werner Maximilian Josef, Dr.
(86) Internationale Anmeldenummer: PCT/EP2000/008385
(87) Internationale Veröffentlichungsnummer: WO 2001/015663

(56) Entgegenhaltungen:
- WO-A-99/02128
- BE-A- 1 000 950
- FR-A- 1 596 818
- FR-A- 2 186 222
- FR-A- 2 397 189
- FR-A- 2 484 836
- FR-A- 2 489 689
- FR-A- 2 498 451
- FR-A- 2 662 078

## Beschreibung

Die vorliegende Erfindung betrifft ein Entschlackungs- und/oder Regenerationsmittel für die externe Anwendung sowie ein Verfahren zur Entschlackung.

Es ist ein altbekanntes Problem, dass wenn der menschliche Körper übersäuert, er sich selbst lebenswichtiger Mineralstoffe beraubt, um die gebildete Säure zu neutralisieren. Endprodukte dieser Vorgänge sind dann Salze (volkstümlich Schlacken genannt), die im Fettgewebe, im Bindegewebe, in Muskeln und Knorpeln, in Knochen und Gelenken, in den Gefäßwänden, in den Körperorganen und zuletzt im Gehirn abgelagert werden. Der betroffene Mensch fühlt sich abgeschlafft und ausgelaugt, da die dauernde Übersäuerung und die damit verbundene Ablagerung der Schlacken ihn erkranken lassen. Fortschreitend treten dann Parodontose und Karies, Cellulite und Haarausfall, Rheuma, Gicht, Arthrose, Leber-, Magen-, Darmbeschwerden, und im späteren Stadium bei fehlender Therapie, Gehörsturz, Schlaganfall, Herzinfarkt und Tod ein.

Zur Beseitigung der Schlacken und zur Regenerierung des Körpers gibt es verschiedene natürliche Mittel und Methoden, z.B. von innen durch Tee, Nahrungsergänzung zur Darmsanierung und zum Aufbau von Mineralstoffdepots von innen, von außen durch Bäder, Wickel und Packungen.

FR-A-2 498 451 offenbart eine Zusammensetzung geeignet als Entschlakungs- oder Regenerationsmittel für die externe Anwendung (Siehe Seite 1, Zeile 20-21), enthaltend ein Mineralpulver aus (Siehe Seite 5, Zeile 1-3):
- Tonerde
- Meersalz und Mineralien aus Meerwasser
- Algen
- ätherischem Öl (essence de cajeput),
jedoch ohne genaue Prozentangaben.

Ein Nachteil insbesondere für die von außen durchgeführte Unterstützung der Entschlackung und Regeneration sind jedoch die Ortsgebundenheit, d.h. bei Einsatz von Bädern ist man in seinen körperlichen Aktivitäten relativ stark eingeschränkt und es tritt auch - insbesondere auch in der heutigen Zeit - ein für viele Personen nicht zulässiger oder annehmbarer Zeitverlust ein. Selbst bei Wickeln ist ein Problem, dass man auch hier an eine ortsfeste Position gebunden ist.

Aufgabe der Erfindung ist es daher, ein Entschlackungs- und/oder Regenerationsmittel zur äußerlichen Anwendung bereitzustellen, dass ortsungebunden angewandt werden kann, und ein Verfahren zur Entschlackung.

Erfindungsgemäß wird diese Aufgabe durch die Merkmale der Ansprüche 1 und 4 gelöst.

Bevorzugte Weiterbildungen ergeben sich aus den Unteransprüchen.

Gemäß der Erfindung besteht das Entschlackungs- und/oder Regenerationsmittel aus einem Mineralpulver aus im wesentlichen Tonerde, unter Zusatz von Meersalz, Mineralien, Algen und ätherischen Ölen. Der Gehalt von Tonerde beträgt 30 bis 60 Gew.-%, der der Meersalze, und der Mineralien 18 bis 45 Gew.-%, der der Algen, als Trockenextrakt, 18 bis 45 Gew.-%, denen 0,01 bis 1,5 Gew.-% ätherische Öle zugesetzt werden in dem gesamten erfindungsgemäßen Mittel. Aufgrund der Tatsache, dass es sich bei den Produkten ausschließlich um Substanzen natürlichen Ursprungs handelt, die frei von schädlichen Nebenprodukten sind, können mit dieser Substanz auch Personen in Kontakt gebracht werden, die eine empfindliche Hautoberfläche haben. Aber selbst wenn es zu einer nur ganz geringen Resorption über die Haut kommt, ist aufgrund des erfindungsgemäßen Mittels eine Schädigung des Körpers nicht gegeben. Auch eine orale Aufnahme in geringen Mengen ist nicht toxisch.

Vorzugsweise soll das erfindungsgemäße Entschlackungs- und/oder Regenerationsmittel basisch sein, wobei ein pH-Wert ab pH > 8,0 vorzugsweise verwendet wird. Damit wird der Effekt des erfindungsgemäßen Mittels aufgrund der Entsäuerung des übersäuerten Körpers durch Schlacken entgegengewirkt, so dass in einer Art Osmose ein Ausgleich des körpereigenen pH-Wertes möglich ist.

Als für die Erfindung besonders geeignete Bestandteile haben sich naturreine Tonerde, der noch etwas Natriumcarbonat, Natriumhydrogencarbonat und als ätherische Öle solche von z.B. Orangen und Lemongras beigemischt sind, gezeigt, wobei die Tonerde innerhalb geringer natürlichen Schwankungen eine einheitliche mineralische Zusammensetzung besitzt und im wesentlichen aus einem Geschiebemergel von Quarz, Feldspat, Kalkspat, lllit, Kaolinit, Dolomit, Glimmer und Eisenoxid aufweist.

Erfindungsgemäß enthält die für die Zwecke der Erfindung eingesetzte Tonerde 35 bis 65 Gew.-%, insbesondere 42 bis 57 Gew.-%, vorzugsweise 47 bis 52 Gew.-% Silicium, berechnet als SiO₂, 10 bis 18 Gew.-%, vorzugsweise 13 bis 15 Gew.% Aluminium, berechnet als AlO₂, 5 bis 11 Gew.-%, vorzugsweise 7 bis 9 Gew.-% Calcium berechnet als CaO, 3 bis 7, vorzugsweise 4 bis 6 Gew.-% Eisen, berechnet als Fe₂O₃, 2,0 bis 6,5 Gew.-%, vorzugsweise 3,0 bis 5,0 Gew.-% Kalium berechnet als K₂O, 1,5 bis 4 Gew.-%, vorzugsweise 2,0 bis 3,0 Gew.-% Magnesium berechnet als MgO, 0,05 bis 0,4 Gew.-%, vorzugsweise 0,1 bis 0,3 Gew.-% Natrium, berechnet als Na₂O, 0,05 bis 0,4 Gew.-%, vorzugsweise 0,1 bis 0,3 Gew.-% Mangan berechnet als MnO und 0,05 bis 0,25 Gew.-%, vorzugsweise 0,1 bis 0,2 Gew.-% Phosphor berechnet als P₂O₅. Zu diesen wesentlichen Bestandteilen kommen noch die Spurenelemente Kupfer, Kobalt, Lithium sowie Molybdän hinzu, wobei die jeweiligen Werte in einer Größenordnung von 20 +/- 10 ppm (Cu), 15 +/-10 ppm (Co), 3 +/- 1 ppm Lithium (Li) sowie < 1 ppm Molybdän (Mo) betragen. Die toxischen Spurenelemente, die üblicherweise den Tonerden der vorstehend genannten Zusammensetzung enthalten sind, liegen bei Quecksilber, Blei und Cadmium deutliche unter 1 ppm.

Bei den vorstehend genannten toxischen Spurenelementen ist dabei insbesondere noch zu berücksichtigen, dass es sich um in Wasser unlösliche Oxide handelt, die auch bei versehentlich oraler Aufnahme durch die salzsäurehaltige Magensäure nur zum geringen Teil gelöst werden. Die tatsächlich resorbierte Menge liegt dabei deutlich unter 1 ppm.

Zu den vorstehend genannten wesentlichen Bestandteilen der Tonerde der Zusammensetzung, die vorzugsweise 45 bis 60 Gew.-% des erfindungsgemäßen Mittels umfaßt, kommen dann noch Meersalz und Mineralien in der vorstehend angegebenen Menge, insbesondere von 20 bis 35 Gew.-%, Meeresalgenextrakte, insbesondere Grünalgen, in der eingangs erwähnten Menge, vorzugsweise von 20 bis 35 Gew.-% hinzu. Die ätherischen Öle die überdies zum Wohlbefinden beim Entschlacken und regenerieren dienen sind im wesentlichen handelsüblich erhältliche ätherische Öle wie Orangenöl, Ylang Ylang, Rosenöle, Fichtennadelextrakte oder dgl., wobei überdies auch noch aufgrund seines erfrischenden Geruches Lemongras hinzugemischt werden kann.

Das erfindungsgemäße Entschlackungs- und/oder Regenerationsmittel wird ohne Zusatz von Hilfsstoffen hergestellt, an der Luft getrocknet und naturbelassen, wobei verstärkt Wert darauf gelegt wird, dass keine Konservierungsstoffe zugesetzt werden bzw. enthalten sind, auch nicht über einen längeren Lagerungszeitraum.

Beim erfindungsgemäßen Verfahren zur Entschlackung wird primär eine Aufschlämmung des erfindungsgemäßen Mittels in einer für die menschlichen Bedürfnisse geeigneten Lösung hergestellt, diese Aufschlämmung nachfolgend in ein in der Nähe der Körperoberfläche zu tragendes Bekleidungsstück eingebracht und anschließend das Bekleidungsstück über einen gewissen Zeitraum getragen. Die Auflösung bzw. Aufschlämmung des erfindungsgemäßen Mittels in Wasser von Raumtemperatur oder auch geringfügig erhöht, ergibt dabei eine basische Lösung mit einem pH-Wert von mehr als 8,5, in der Regel pH 8,5 bis 9,2.

Als für die Zwecke der Erfindung geeignete Menge hat es sich dabei gezeigt, dass das erfindungsgemäße Mittel in eine Menge von 0,05 bis 2,5 Gew.-%, vorzugszweise 1,0 bis 2,0 Gew.-% pro Anwendung in ein Lösungsmittel eingebracht werden, wobei die Menge des zu verwendenden Lösungsmittels, vorzugsweise Wasser, davon abhängt, ob eine Tränkung oder eine Besprühung des damit zu behandelnden Bekleidungsstückes als ausreichend angesehen wird.

Vorzugsweise werden ein aus einem Ober- und einem Unterteil bestehender Anzug verwendet, dessen Innenteile die wegen ihrer Hautverträglichkeit vorzugsweise aus reiner Baumwolle sind, direkt auf der Haut getragen werden, nachdem die Teile innen mit der auf Raumtemperatur oder handwarm angerührten basischen Lösung aus dem erfindungsgemäßen Mittel besprüht oder getränkt worden sind. Aufgrund osmotischer Wirkung entzieht dabei die Lösung dem Körper die sauren Schlackenstoffe und tritt gleichzeitig eine Remineralisierung ein.

Über diesen Anzug ist es möglich, einen atmungsaktiven Überanzug zu tragen, der hauteng und elastisch am Körper anliegt, so dass die Person beweglich bleibt und sogar Arbeiten oder Gymnastik möglich ist, während die basische Lösung ihre Wirkung entfaltet.

Je nach dem, wie intensiv und konsequent und über welchen Zeitraum das erfindungsgemäße Entschlackungsverfahren mit dem erfindungsgemäßen Entschlakkungsmittel angewendet, auch zusätzlich die im Alltag ermittelten Giftzufuhren reduziert oder vermieden und die Ernährung verbessert werden, desto schneller und umfassender wird sich auch der Körperumfang reduzieren, die Haut sich fester und glatter anfühlen und sich somit ein allgemeines Wohlbefinden und ein positives Lebensgefühl einstellen.

## Patentansprüche

1. Entschlackungs- und/oder Regenerationsmittel für die externe Anwendung enthaltend ein Mineralpulver aus 30 bis 60 Gew.-% Tonerde, 18 bis 45 Gew.-% Meersalz und Mineralien, 18 bis 45 Gew.-% Algen und 0,01 bis 1,5 Gew.-% ätherisches Öl.

2. Entschlackungsmittel nach Anspruch 1,
**dadurch gekennzeichnet, dass**
das Mittel basisch ist.

3. Entschlackungsmittel nach einem der Ansprüche 1 oder 2,
**dadurch gekennzeichnet, dass**
die Tonerde 35 bis 65 Gew.-% Silicium berechnet als SiO₂, 13 bis 15 Gew.-% Aluminium, berechnet als AlO₂, 7 bis 9 Gew.-% Calcium, berechnet als CaO, 4,0 bis 6,0 Gew.-% Eisen, berechnet als Fe₂O₃, 3,0 bis 5,0 Gew.-% Kalium, berechnet als K₂O, 2,0 bis 3,0 Gew.-% Magnesium, berechnet als MgO, 0,1 bis 0,3 Gew.-% Natrium, berechnet als Na₂O, 0,1 bis 0,3 Gew.-% Mangan, berechnet als MnO, und 0,1 bis 0,2 Gew.-% Phosphor, berechnet als P₂O₅ enthält.

4. Kosmetisches Verfahren zur Entschlackung und/oder Regeneration
**dadurch gekennzeichnet, dass**
a) eine Aufschlämmung eines Entschlackungs- und/oder Regenerationsmittels nach einem der Ansprüche 1 bis 3 hergestellt,
b) die Aufschlämmung in ein in der Nähe der Körperoberfläche zu tragendes Bekleidungsstück eingebracht und
c) das Bekleidungsstück getragen werden.

5. Entschlackungskitt bestehend aus einem Bekleidungsstück, einem Entschlackungs- und/oder Regenerationsmittel nach einem der Ansprüche 1 bis 3 sowie einer Dosiereinrichtung.

## Claims

1. Purifying and/or regenerating agent for external use comprising a mineral powder consisting of 30 to 60% by weight of argillaceous earth, 18 to 45% by weight of sea salt and minerals, 18 to 45% by weight of algae and 0.01 to 1.5% by weight of essential oil.

2. Purifying agent according to claim 1, **characterized in that** the agent is basic.

3. Purifying agent according to one of claims 1 or 2, **characterized in that** the argillaceous earth comprises 35 to 65% by weigh silicon, calculated as SiO₂, 13 to 15% by weight aluminium, calculated as AlO₂, 7 to 9% by weight calcium, calculated as CaO, 4.0 to 6.0% by weight iron, calculated as Fe₂O₃, 3.0 to 5.0% by weight potassium, calculated as K₂O, 2.0 to 3.0% by weight magnesium, calculated as MgO, 0.1 to 0.3% by weight sodium, calculated as Na₂O, 0.1 to 0.3% by weight manganese, calculated as MnO, and 0,1 to 0,2% by weight phosphorus, calculated as P₂O₅.

4. Cosmetic method for purifying and/or regeneration organism, **characterized in that**
a) a suspension of a purifying and/or regenerating agent according to one of claims 1 to 3 is prepared,
b) the suspension is put in a clothing wom close to the surface of the body and
c) the clothing piece is worn.

5. Purifying kit consisting of a clothing, a purifying and/or regenerating agent according to one of claims 1 to 3, and a metering device.

## Revendications

1. Agent d'élimination et/ou de régénération pour l'usage externe contenant une poudre minérale constituée de 30 à 60 % en poids d'alumine, 18 à 45 % en poids de sel de mer et de minéraux, 18 à 45 % en poids d'algues et 0,01 à 1,5 % en poids d'huile essentielle.

2. Agent d'élimination selon la revendication 1, **caractérisé en ce que** l'agent est basique.

3. Agent d'élimination selon l'une des revendications 1 ou 2, **caractérisé en ce que** l'alumine contient 35 à 65 % en poids de silicium, calculé en tant que SiO₂, 13 à 15 % en poids d'aluminium, calculé en tant que AlO₂, 7 à 9 % en poids de calcium, calculé en tant que CaO, 4,0 à 6,0 % en poids de fer, calculé en tant que Fe₂O₃, 3,0 à 5,0 % en poids de potassium, calculé en tant que K₂O, 2,0 à 3,0 % en poids de magnésium, calculé en tant que MgO, 0,1 à 0,3 % en poids de sodium, calculé en tant que Na₂O, 0,1 à 0,3 % en poids de manganèse, calculé en tant que MnO, et 0,1 à 0,2 % en poids de phosphore, calculé en tant que P₂O₅.

4. Procédé cosmétique d'élimination et/ou de régénération, **caractérisé en ce que**
a) une décantation d'un agent d'élimination et/ou de régénération selon l'une des revendications 1 à 3 est produite,
b) la décantation est appliquée sur une pièce de vêtement à porter à proximité de la surface du corps et
c) la pièce de vêtement est portée.

5. Kit d'élimination constitué d'une pièce de vêtement, d'un agent d'élimination et/ou de régénération selon l'une des revendications 1 à 3 ainsi que d'un dispositif doseur.
